Europäisches Patentamt

European Patent Office

Office européen des brevets

(11) Veröffentlichungsnummer: **0 352 412 B1**

(12) **EUROPÄISCHE PATENTSCHRIFT**

(45) Veröffentlichungstag der Patentschrift: **12.01.94**

(21) Anmeldenummer: **89107645.7**

(22) Anmeldetag: **27.04.89**

Die Akte enthält technische Angaben, die nach
dem Eingang der Anmeldung eingereicht wurden
und die nicht in dieser Patentschrift enthalten sind.

(51) Int. Cl.⁵: **A61K 9/72**, A61K 31/23,
A61K 31/22

(54) **Ester des Retinols und/oder der Retinsäure enthaltende Aerosol-Inhalate.**

(30) Priorität: **04.05.88 DE 3815221**

(43) Veröffentlichungstag der Anmeldung:
**31.01.90 Patentblatt  90/05**

(45) Bekanntmachung des Hinweises auf die
Patenterteilung:
**12.01.94 Patentblatt  94/02**

(84) Benannte Vertragsstaaten:
**AT BE CH DE ES FR GB GR IT LI LU NL SE**

(56) Entgegenhaltungen:
**EP-A- 0 009 793**
**FR-M- 6 419**
**US-A- 3 584 115**
**US-A- 3 957 966**
**US-A- 4 525 341**

**CHEMICAL ABSTRACTS, Band 73, 1970, Seite
216, Zusammenfassung Nr. 59219p, Columbus, Ohio, US; H. VALFRE: "Stability of vitamin A in cosmetic aerosol formulations"**

**J.E.F. REYNOLDS: "Martindale - The Extra
Pharmacopoeia", Ausgabe 28, 1982, Seiten
1635-1638, The Pharmaceutical Press, Lon-**

**don, GB**

(73) Patentinhaber: **HERMES Fabrik pharmazeutischer Präparate Franz Gradinger GmbH &
Co.
Postfach 2 09
D-82043 Grosshesselohe/München(DE)**

(72) Erfinder: **Biesalski, Hans Konrad, Dr.med.
Alzeyer Pforte 2
D-6509 Albig(DE)**

(74) Vertreter: **Reinhard, Skuhra, Weise
Postfach 44 01 51
D-80750 München (DE)**

EP 0 352 412 B1

## Beschreibung

Die Erfindung betrifft eine pharmazeutische Zubereitung.

Der Begriff Vitamin A steht für eine Reihe chemisch ähnlicher Verbindungen mit unterschiedlicher Wirkung im menschlichen und tierischen Organismus. Dazu gehören als wesentliche Gruppen der Alkohol des Vitamins, das Retinol - die Transportform des Vitamins im Blut -, der Aldehyd - der biologisch aktive Metabolit beim Sehvorgang -, der Retinylester - die Speicherform des Vitamins in der Leber und in Schleimhäuten und Keimdrüsen - sowie die Retinsäure mit verschiedenen Derivaten, die vor allem bei den Differenzierungsvorgängen der Haut eine Rolle spielt. Das Vitamin ist für den Menschen essentiell, d.h. es entsteht ein Vitamin-Mangel, wenn es nicht mit der Nahrung zugeführt wird. Der Vitamin-A-Mangel äußert sich je nach Dauer der ausbleibenden Zufuhr in Verhornung der Schleimhäute (Biesalski, H.K. Stofft, E., Wellner U., Niederauer, U. and Bässler, K.H. Vitamin A and ciliated cells. I. Respiratory epithelia Z.f. Ernähungswissenschaft: 25, 114-122 (1986) sowie McDowell, E.M., Keenan K.P., Huang M.: Effect of Vitamin A deprivation on Hamster, Brachial Epithelium, Virchow's Arch. (Cell.Pathol.), 45: 197-219, 1984 und McDowell E.M., Keenan K.P., Huang M.; Restoration of mucociliary Brachial Epithelium following Deprivation of Vitamin A, Virchow's Arch. (Cell. Pathol.) 45: 221-240, 184) vor allem der Atmungswege, erhöhter Infektanfälligkeit sowie bei ausgeprägtem Mangel in den klassischen Symptomen der Verhornung der Bindehaut des Auges bis zur vollständigen Zerstörung und Blindheit.

Bei Zufuhr des Vitamins sind die meisten mangelbedingten Veränderungen vor allem im Bereich der Schleimhäute rückbildungsfähig. Diese typischen durch den Vitamin-A-Mangel bedingten Veränderungen finden sich in ähnlicher Weise bei Erkrankungen der Schleimhäute der Atmungswege, wie sie durch akute und chronische Bronchitis, Nikotininhalation und auch frühe krebsartige Veränderungen verursacht werden. An tierexperimentellen Untersuchungen wie auch beim Menschen konnte gezeigt werden, daß solche nicht durch Vitamin-A-Mangel bedingten Veränderungen durch systemische Zufuhr des Vitamins meist in hoher Dosierung rückgängig gemacht werden konnten. Vor allem bei verschiedenen Krebsformen ist der Einsatz einer hochdosierten systemischen Vitamin-A-Therapie im Sinne einer Rezidivprophylaxe nach Primärbehandlung des Tumors mit unterschiedlichem Erfolg beschrieben.

Bei der systemischen Anwendung müssen zur Erreichung einer Rückbildung der Plattenepithelmetaplasien oder zur Verhinderung eines erneuten Auftretens solcher Veränderungen hohe Konzentrationen eingesetzt werden, die zu teilweise erheblichen Nebenwirkungen (Hirndrucksymptomatik, Leberzellstoffwechselstörungen, u.a.) führen. Daneben besteht eine ausgeprägte teratogene Wirkung im ersten Trimenon, die einen Einsatz hochdosierter Therapie in der Schwangerschaft verbietet (Bauernfeind JC. (1980): The safe use of vitamin A, The nutrition foundation, Washington DC).

Des weiteren ist eine Zufuhr von Vitamin A auch in physiologischer Konzentration bei Störungen des Leberzellstoffwechsels wie z.B. Entzündung oder Zirrhose nicht zulässig, da durch die gleichzeitig gestörte Proteinsynthese (mangelnde Bildung des Transportproteins - RBP = Retinol bindendes Protein) der Leber eine Ausschleusung des Vitamins aus den Speichern, in die es nach der Resorption überführt wird, nicht möglich ist und dadurch eine weitere Schädigung der Leber eintreten würde (Smith, F.R. and Goodman, D.S. The effect of diseases of the liver, thyroid, and kidneys on the transport of vitamin A in human plasma. J. Clin. Invest.: 50, 2426 (1971); Weber, F.L. Mitchell, G.E., Powell, D.B., Reiser, B.J. and Banwell, J.G. Reversible hepatoxicity associated with hepatic vitamin A accumulation in a protein deficient patient. Gastroenterology: 82, 118-123 (1982)). Hinzu kommt, daß durch die peripheren Zielgewebe, wie z.B. das Respirationsepithel, das Vitamin nur dann aufgenommen und seiner Funktion zugeführt werden kann, wenn es an eben dieses Transportprotein gebunden ist.

Aus der EP-A-0 009 793 sind 2-(Retinyliden)-malondinitrilester bekannt. Die hier beschriebenen neuen, nicht physiologischen chemischen Substanzen sollen eine verminderte Toxizität und eine günstigere Pharmakokinetik aufweisen. Hierdurch soll eine Alternative zu den bekannten, toxischen Vitamin-A-Verbindungen (Retinsäure und Ester des Retinols) bereitgestellt werden. Wie auf Seite 5, letzter Absatz der EP 0 009 793 beschrieben wird, werden diese neuen und physiologischen Substanzen entweder oral appliziert oder äußerlich in Form von Sprays verwendet. Die Formulierung der natürlich vorkommenden Ester der Retinsäure und des Retinols in Form eines Aerosol-Inhalats wird hierdurch nicht nahegelegt.

In der US 4 525 341 wird ein System zur Applikation von Vitaminen, z.B. von Vitamin A, in Form eines Mund- oder Nasensprays beschrieben. Hierdurch sollen die Vitamin-Präparationen auf die Schleimhäute des Nasen- oder Mundraumes aufgesprüht werden, um von hier entweder durch den Abtransport mit dem Schleimhautsekret, d.h. mit dem Speichel, in den Gastrointestinaltrakt aufgenommen zu werden. Derartige pharmazeutische Zubereitungen entsprechen pharmakokinetisch und pharmakodynamisch der konventionellen oralen Verabreichung üblicher Vitaminpräparate, z.B. in Form von Saft, Tabletten und Lutschpastillen, zur Ergänzung der Vitaminzufuhr mit der Nahrung. Eine Zubereitungsform als Spray soll damit nur solchen Menschen, die Schwierigkeiten mit der Einnahme von Tabletten haben, eine bequem und einfach zu handhabende Alternativlösung bieten. Die in der US 4 525 341 offenbarten Aerosole sind gemäß der US-Pharmacopeia keine Aerosol-Inhalate. Die Inhalation von Aerosolen, wie sie in der US 4 525 341 beschrieben sind, ist gemäß der US-Pharmacopeia kontraindiziert.

In der US 3 584 115 wird ein Aerosolsystem beschrieben, dessen Besonderheit darin besteht, das unmittelbar nach dem Aufsprühen auf einen Untergrund, z.B. auf die Haut, dort ein weißer Film sichtbar wird, der eine visuelle Kontrolle ermöglicht, welches Areal beim Sprühen erreicht wurde, und der wenig später verschwindet. In Spalte 1, Zeilen 41 - 56 ist ausschließlich die Anwendung auf der Haut genannt. Eine Anwendung auf Schleimhäuten in Form von Aerosol-Inhalaten der in der US 3 584 115 offenbarten Zubereitung ist schlechterdings unmöglich.

Eine Aufgabe der vorliegenden Erfindung ist es, eine Retinoide enthaltende pharmazeutische Zubereitung zur Prophylaxe und Behandlung von Erkrankungen der Schleimhäute des Tracheo-Bronchialtraktes, insbesondere des tiefen Bronchialtraktes bereitzustellen.

Diese Aufgabe wird erfindungsgemäß gelöst durch eine pharmazeutische Zubereitung, die besteht aus einem Ester der Retinsäure und/oder einem Ester des Retinols als Wirkstoffe, die in der Zubereitungsform eines Aerosol-Inhalats vorliegen. Hierdurch wird insbesondere eine topische Einwirkung des Wirkstoffs auf die Schleimhäute ermöglicht.

Insbesondere sind erfindungsgemäß vorteilhaft einzusetzen Ester des Retinols mit physiologisch unbedenklichen Carbonsäuren und Ester der Retinsäure mit physiologisch ununbedenklichen Alkoholen.

Als Carbonsäuren zur Herstellung der Retinolester sind erfindungsgemäß insbesondere vorteilhaft einzusetzen die gesättigten und ungesättigten Fettsäuren, insbesondere die endogen vorkommenden Fettsäuren.

Ganz besonders vorteilhaft sind Palmitinsäure und/oder Stearinsäure und/oder Ölsäure und/oder Linolsäure und/oder Linolensäure.

Als Alkohole zur Herstellung der Retinsäureester sind erfindungsgemäß insbesondere vorteilhaft einzusetzen einwertige und/oder mehrwertige primäre und/oder sekundäre und/oder tertiäre aliphatische und/oder alicyclische und/oder aromatische Alkohole.

Ganz besonders vorteilhaft einzusetzen sind die aliphatischen, einwertigen primären Alkohole, insbesondere Methanol und Ethanol und die Fettalkohole.

Als Aerosole sind erfindungsgemäß insbesondere einsetzbar Sprays und Inhalate, insbesondere tiefenwirksame Inhalate.

Erfindungsgemäß ist erreicht, daß der Wirkstoff in ein Aerosol überführt ist, so daß die feinverteilten winzigen Wirkstoffpartikeln des Aerosols an den Wirkort z.B. die Schleimhaut des Respirationstraktes gelangen.

Unter Aerosolen bzw. Sprays werden erfindungsgemäß disperse Systeme aus Gasen mit darin verteilten festen oder flüssigen Teilchen von etwa $10^{-7}$ bis $10^{-1}$cm Durchmesser verstanden. Im Hinblick auf die erfindungsgemäße pharmazeutische Zubereitung sind daher zu unterscheiden:

A) Verteilungen fester Wirkstoffpartikel im Trägergas (Staubaerosole) und

B) Verteilungen einer wirkstoffhaltigen Flüssigkeit im Trägergas (Nebelaerosole).

Die sogenannten Nebelaerosole wiederum können vorliegen als

a) Verteilung des in einem nicht wässerigen, lipophilen, leicht flüchtigen und physiologisch unbedenklichen Lösungsmittel gelösten Wirkstoffes im Trägergas oder als

b) Verteilung einer Lösung bzw. Dispersion des Wirkstoffes bzw. einer Emulsion des in flüssiger Form vorliegenden Wirkstoffes in Wasser oder Gemischen aus Wasser und/oder mit Wasser mischbaren physiologisch unbedenklichen Flüssigkeiten im Trägergas.

Für die technische Ausführung von Präparationen, die zur Applikation eines erfindungsgemäßen Wirkstoffes in Form einer Spray- oder Aerosol-Verteilung dienen, sind unter anderem folgende an sich bekannte technologischen Systeme möglich:

1. Pumpzerstäuber:

Mittels eines Kolbenpumpmechanismus im Sprühkopf wird in dem die Wirkstofflösung oder -Dispersion enthaltenden Zerstäubergefäß ein Überdruck erzeugt, wodurch die wirkstoffhaltige Flüssigkeit durch die Zerstäuberdüse gepreßt und dabei in der Raumluft verteilt wird (vorzugsweise geeignet für Aerosol des Typs Bb).

2. Aerosol-Treibgaspackungen:

Ein Treibgas sorgt für den Überdruck im Behälter, aus dem durch Öffnen des Verschlußventiles der suspendierte oder gelöste Wirkstoff durch die Zerstäuberdüse gepreßt und verteilt wird. Zu unterscheiden sind dabei:

a) Flüssiggassysteme:

Als Treibgas dient ein verflüssigtes Gas (z.B. niedrig siedende FCKW oder Propan, Butan), wobei das System ausgelegt sein kann als

aa) Zweiphasen-Aerosol:

Dabei liegt das Treibgas im Druckbehältnis in der flüssigen und gasförmigen Phase vor, wobei die flüssige Phase gleichzeitig den Wirkstoff sowie eventuelle Hilfsstoffe, wie zusätzliche Lösungsmittel, gelöst enthält (vorzugsweise geeignet für Aerosole des Typs Ba).

ab) Suspensionsaerosol:

Dabei sind die Wirkstoffpartikel in fester Form in der flüssigen Treibmittelphase suspendiert (vorzugsweise geeignet für Aerosole des Typs A).

ac) Dreiphasen-Aerosol:

Zur gasförmigen und flüssigen Treibmittelphase tritt noch eine flüssige, mit dem Treibmittel nicht mischbare Phase der Wirkstofflösung. Im Gegensatz zu 2aa) und 2ab) tritt beim Sprühvorgang, solange die Packung noch Wirkstoffphase enthält kein Treibmittel mit aus, das in diesem Fall alleine zur Erzeugung des Betriebsdruckes in der Packung dient (vorzugsweise geeignet für Aerosole des Typs B).

b) Druckgassystem:

Bei diesem tritt anstelle des verflüssigten Gases ein komprimiertes Gas (z.B. Stickstoff, Kohlendioxid, Distickstoffmonoxid, Luft). Beim Öffnen des Ventils wird die Wirkstoffphase über die in sie eintauchende Steigleitung durch das Zerstäuberventil gepreßt. Das Treibgas tritt dabei nur in dem Umfang nach außen, in dem es in der Wirkstoffphase löslich ist; bei Verwendung einer Zweikammerdruckpackung ist dies jedoch überhaupt nicht der Fall, da sich die Wirkstoffphase in einem separaten Plastiksack in der Druckdose befindet und mit dem Treibgas nicht in direktem Kontakt steht (vorzugsweise geeignet für Aerosol des Typs B).

Die Herstellung der erfindungsgemäßen pharmazeutischen Zubereitung geschieht also erfindungsgemäß, indem der Wirkstoff in einem geeigneten untoxischen Medium gelöst oder dispergiert wird und diese Lösung oder Dispersion zu einem Aerosol zerstäubt, d.h. in einem Trägergas feinst verteilt wird. Dies ist technisch möglich, z.B. in Form von Aerosol-Treibgaspackungen, Pumpaerosolen oder sonstigen an sich bekannten Apparaturen zur Flüssigkeitsvernebelung und Feststoffvernebelung, die insbesondere eine genaue Einzeldosierung erlauben.

Das Trägergas kann erfindungsgemäß Teil der pharmazeutischen Zubereitung sein in Form eines Treibgases oder eines beim Zerstäuben verdampfenden Lösungsmittels, wie im Falle der Aerosol-Treibgaspackung. Das Trägergas kann aber auch Raumluft sein, die zum Zwecke der Zerstäubung der wirkstoffhaltigen Flüssigkeit z.B. als kräftiger Luftstrom über die Öffnung eines in die Flüssigkeit eintauchenden Kapillarröhrchens geleitet wird, durch welches die Lösung dabei angesaugt und dann in dem Trägergasstrom zerteilt wird. Dieser Trägergasstrom kann beispielsweise durch einen in die Zerstäuberapparatur integrierten Kompressor erzeugt werden. Schließlich kann als Trägergas auch die ruhende Umgebungsluft dienen, in welcher die feinen Tröpfchen der wirkstoffhaltigen Flüssigkeit verteilt werden, nachdem sie z.B. in der Zerstäuberdüse eins Pumpsprays, Dreiphasenaerosols oder einer Zweikammer-Druckpackung erzeugt wurden.

Eine andere Möglichkeit der Zerstäubung ist eine mechanische Zerteilung der den Wirkstoff bzw. die Wirkstoffe enthaltenden Flüssigkeit, z.B. durch Ultraschallwellen z. B. mit Hilfe eines handelsüblichen Ultraschallinhalators.

Der Wirkstoff bzw. die Wirkstoffkombination wird vorzugsweise in stabilisierter Form eingesetzt; dies wird beispielsweise durch Zugabe von Butylhydroxyanisol (BHA) und/oder Butylhydroxytoluol (BHT) oder gamma-Tocopherol oder alpha-Tocopherol oder Tocopherolgemischen oder von anderen Antioxidantien bzw. von deren Gemischen erreicht.

Die Stabilisierung erfolgt unabhängig von der Art des eingesetzten Vitamin-A-Derivates.

Die Wirkstoffe können erfindungsgemäß entweder als feste Teilchen im Trägergas oder in gelöster Form im Trägergas vorliegen.

Für den Fall, daß die Wirkstoffe in gelöster Form im Trägergas vorliegen, sind erfindungsgemäß zwei Möglichkeiten vorgesehen, nämlich eine Wirkstofflösung in einem nichtwäßrigen flüchtigen Lösungsmittel oder eine Wirkstofflösung in einem wäßrigen Lösungsmittel.

Die Lösung des Wirkstoffs kann erfindungsgemäß vorzugsweise in einem nichtflüchtigen, nichttoxischen, physiologisch verträglichen Lösungsmittel erfolgen.

Besonders vorteilhaft ist eine Dosieraerosol-Treibgaspackung zu verwenden. Zu ihrer Herstellung wird der Wirkstoff zunächst in einem toxikologisch unbedenklichen, inerten Lösungsmittel gelöst; hierzu eignen sich insbesondere Fluorchlorkohlenwasserstoffe wie 1,1,2-Trichlor-1,2,2-Trifluorethan (Frigen 13), das gleichzeitig noch als eine Komponente der Treibgasmischung wirkt. Die Lösung wird dann in ein druckfestes Behältnis eingebracht z.B. in eine mit einem Innenschutzlack versehene Aluminiumdose. Danach wird ein Treibgas bzw. Treibgasgemisch, bestehend aus verflüssigten oder komprimierten Gasen, z.B. ein Gemisch aus niedrigsiedenden Fluorchlorkohlenwasserstoffen, nach Entfernen der Luft aus der Dose in diese unter Druck eingebracht und die Dose mit einem Dosierventil geschlossen. Die Komponenten des Treibgases sind toxikologisch unbedenklich und inert; geeignet sind beispielsweise Dichlordifluormethan (Frigen 12) oder 1,2-Dichlor-1,1,2,2-Tetrafluorethan (Frigen 114) oder verflüssigte Gase, wie Propan, Butan oder Dimethylether.

Neben diesem Verfahren der sog. Druckabfüllung sind erfindunggemäß beispielsweise auch das Kälteverfahren oder das "under the cup filling"-Verfahren möglich.

Die auf diese Weise hergestellte erfindungsgemäße pharmazeutische Zubereitung besteht aus:

0,01 - 50 Gew.% Wirkstoff,
0 - 49,99 Gew.% Lösungsmittel und
50 - 99,99 Gew.% Treibgas.

Vorzugsweise besteht sie aus

0,01 - 30 Gew.% Wirkstoff
0 - 30 Gew.% Lösungsmittel und
40 - 99,99 Gew.% Treibgas.

Insbesondere sind geeignet Zubereitungen aus:

0,05 - 25 Gew.% Wirkstoff,
0 - 30 Gew.% Lösungsmittel und
45 - 99,95 Gew.% Treibgas.

Vorteilhaft sind Zubereitungen aus:

0,1 - 15 Gew.% Wirkstoff,
0 - 25 Gew.% Lösungsmittel und
60 - 99,9 Gew.% Treibgas.

Insbesondere vorteilhaft sind Zubereitungen aus

0,1 - 10 Gew.% Wirkstoff,
0 - 25 Gew.% Lösungsmittel und
65 - 99,9 Gew.% Treibgas.

Als besonders vorteilhaft haben sich erwiesen Zubereitungen aus:

0,1 - 7 Gew.% Wirkstoff,
5 - 23 Gew.% Lösungsmittel und
70 - 94,9 Gew.% Treibgas.

Besonders vorteilhaft zu verwenden sind auch Zubereitungen aus:

0,1 - 5 Gew.% Wirkstoff,
10 - 20 Gew.% Lösungsmittel und
75 - 89,9 Gew.% Treibgas.

Hervorragende Ergebnisse liefert eine Zubereitung aus:

0,17 Gew.% Wirkstoff,
19,83 Gew.% Lösungsmittel und
80 Gew.% Treibgas
zur Applikation niedriger Einzeldosen des Wirkstoffes und Zubereitungen aus
3,9 Gew.% Wirkstoff,
20 Gew.% Lösungsmittel und
76,1 Gew.% Treibgas
zur Applikation hoher Einzeldosen des Wirkstoffes.

Die Wirkstoffgehalte der beiden letzten Zubereitungen beziehen sich auf Retinolpalmitat (1,7 Mio. I.E./g). Bei Verwendung anderer Wirkstoffe oder Wirkstoffgemische ist der gewichtsprozentuale anteil so zu korrigieren, daß die in der betreffenden Wirkstoffmenge enthaltene Vitamin-A-Aktivität (ausgedrückt in I.E.) bzw. Vitamin-A-Äquivalentdosis (auf molarer Basis) unverändert bleibt. Die sich daraus ergebenden Zusammensetzungsverschiebungen werden durch entsprechende Korrektur des gewichtsprozentualen Lösungsmittelanteils an der Zubereitung ausgeglichen.

Die vorzugsweise Ausbringmenge der verwendeten Dosierventile liegt z.B. zwischen 35 mg und 100 mg pro Sprühstoß.

Weitere Merkmale der Erfindung ergeben sich aus der folgenden Beschreibung von nicht beschränkenden Ausführungsbeispielen:

Beispiel 1:

| Retinolpalmitat (1,7 Mio. I.E./g), stabilisiert mit BHA/BHT | 3,9 Gew.% |
|---|---|
| 1,1,2-Trichlor-1,2,2-Trifluorethan | 26,1 Gew.% |
| Treibgasgemisch bestehend aus 40 Gew.% Dichlordifluormethan und 60 Gew.% 1,2-Dichlor-1,1,2,2-Tetrafluorethan | 70,0 Gew.% |

Zur Herstellung der Wirkstofflösung werden 13,0 kg Retinolpalmitat (1,7 Mio I.E./g) in 87,0 kg Trichlortrifluorethan gelöst. Die Lösung erfolgt in einem geschlossenen Behälter mit eingebautem Rührwerk, damit keine Verdampfung des Lösungsmittels erfolgen kann. Nach dem Auflösen wird die Flüssigkeit durch einen 50 µm Filter filtriert und die dabei verdampfte Menge an Lösungsmittel wieder ersetzt. Zum Abfüllen wird die klare Wirkstofflösung aus einem dicht geschlossenen Behälter mit einer Entnahmeleitung mittels einer mechanischen Kolbendosiereinrichtung in die dafür vorgesehenen Aerosoldosen eingefüllt. Die Dosiereinrichtung wird so fixiert, das sie immer die gleiche Menge von 4,5 g pro Dosierhub abgibt. Danach wird über die Öffnung der Dose das Aerosoldosierventil gesteckt und mittels einer automatischen Crimpzange das Ventil auf der Aerosoldose unabnehmbar befestigt (gecrimpt). Die so verschlossene Dose wird nun über eine automatische Treibgasfüllanlage durch das Ventil hindurch mit dem betreffenden Treibgasgemisch befüllt. Die Füllmenge für 4,5 g Wirkstofflösung beträgt 10,5 g Treibgas. Das verwendete Dosierventil ist vorteilhaft für eine Ausbringung von 75 mg pro Sprühstoß ausgelegt.

Nach der Prüfung der Dosen auf Dichtigkeit gemäß den technischen Regeln Gase (TRG 402) werden diese mit dem Sprühkopf oder einer entsprechenden Zerstäubereinrichtung versehen, die auf den Ventilkegel des Dosierventils aufgesetzt werden. Zum Schutz der Zerstäubereinrichtung vor Betätigung und Beschädigung wird die Dose noch mit einer geeigneten Schutzkappe versehen oder in eine schützende Umhüllung verpackt.

Beispiel 2:

| Retinolpalmitat (1,7 Mio. I.E./g), stabilisiert mit BHA/BHT | 0,17 Gew.% |
|---|---|
| 1,1,2-Trichlor-1,2,2-Trifluorethan | 19,83 Gew.% |
| Treibgasgemisch aus 40 Gew.% Dichlordifluormethan und 60 Gew.% 1,2-Dichlor-1,1,2,2-Tetrafluorethan | 80,0 Gew. |

Das Dosierventil ist vorteilhaft für eine Ausbringung von 35 mg pro Sprühstoß ausgelegt.

Beispiel 3:

| Retinollaurat, stabilisiert mit alpha-Tocopherol | 0,14 Gew.% |
|---|---|
| Treibgasgemisch bestehend aus 40 Gew.% Chlortrifluorethylen und 60 Gew.% Dichlorfluormethan | 99,86 Gew.% |

Das Dosierventil ist vorteilhaft für eine Ausbringung von 35 mg pro Sprühstoß ausgelegt.

Beispiel 4:

| Retinsäure-Ethylester | 9,2 Gew.% |
|---|---|
| Trichlorfluormethan | 20,8 Gew.% |
| Treibgasgemisch bestehend aus 45 Gew.% 1,1-Difluorethan und 55 Gew.% 1,1-Dichlor-1,2,2,2-Tetrafluorethan | 70 Gew.% |

Das Dosierventil ist vorteilhaft für eine Ausbringung von 75 mg pro Sprühstoß ausgelegt.

Beispiel 5:

Wirkstoffgemisch (stabilisiert mit alpha-Tocopherol) bestehend aus:

| | |
|---|---|
| Retinsäure-Caprylester | 1,15 Gew.% |
| Retinolacetat | 1,40 Gew.% |
| 1,1,2-trichlor-1,2,2-Trifluorethan | 22,45 Gew.% |
| Treibgasgemisch bestehend aus: 40 Gew.% 1,1-Difluorethan und 60 Gew.% 1,1-Dichlor-1,2,2,2-Tetrafluorethan | 75,0 Gew.% |

Das Dosierventil ist vorteilhaft für eine Ausbringung von 75 mg pro Sprühstoß ausgelegt.

Beispiel 6:

Wirkstoffgemisch (stabilisiert mit gamma-Tocopherol) bestehend aus:

| | |
|---|---|
| Retinolpropionat | 0,05 Gew.% |
| Retinololeat | 0,085 Gew.% |
| Trichlorfluormethan | 19,865 Gew.% |
| Propan | 80,0 Gew.% |

Beispiel 7:

```
        Retinolacetat, stabilisiert mit


    gamma-Tocopherol                    22      Gew.%

    1,1,2 Trichlor-1,2,2-Trifluorethan  22      Gew.%

    Treibgasgemisch bestehend aus

    50 % 1-Chlor-1,1-Difluorethan und

    50 % Tetrafluormethan               56      Gew.%
```

Das Dosierventil ist vorteilhaft für eine Ausbringung von 35 mg pro Sprühstoß ausgelegt.

Die Herstellungsweise ist für die Beispiele 2 bis 6 analog der unter Beispiel 1 beschriebenen Verfahrensweise. Im Falle des Beispiels 3 dient ein Drittel des im Treibgasgemisch enthaltenen Trichlorfluormethans zum Auflösen des Wirkstoffes.

Der durch einen Sprühstoß des Dosierventils ausgebrachte Wirkstoffnebel wird durch geeignete Applikatoren in die Körperhöhlen und auf deren Schleimhäute verbracht. So kann er beispielsweise durch ein auf die Sprühdose aufgesetztes Mundstück in die Mundhöhle eingebracht werden. Von dort gelangen beim Einatmen nach dem Sprühvorgang Partikel mit 0,5 - 5 $\mu$m bis in die Lungenalveolen, während Partikel mit 5 - 30 $\mu$m auf die Schleimhäute der oberen Luftwege transportiert werden.

Weitere erfindungsgemäße pharmazeutische Zubereitungen sind solche, bei denen die Wirkstoffe in wäßrigen Lösungsmitteln gelöst oder dispergiert sind. Diese können als "wäßrige Systeme" bezeichnet werden. Unter "wäßrigen Systemen" sind solche Systeme zu verstehen, die als Lösungsmittel Wasser oder Gemische aus Wasser mit anderen physiologisch verträglichen Lösungsmitteln enthalten.

Dem Lösungsmittel Wasser können vorzugsweise dem Fachmann an sich bekannte Hilfsstoffe wie Emulgatoren und/oder Lösungsvermittler zugesetzt werden.

7

Die so hergestellte wirkstoffhaltige Flüssigkeit kann vorzugsweise zur Anwendung kommen

a. In Form einer Dreiphasenflüssiggasaerosolpackung:

Es werden die gleichen Treibgassysteme verwendet wie beim oben beschriebenen Zweiphasenaerosol.

b. In Form eines Druckgasaerosoles:

Die Flüssigkeit wird in eine Sprühdose abgefüllt, die mittels eines komprimierten Gases (z.B. Stickstoff oder Kohlendioxid) unter Druck gesetzt wird.

c. In Form eines Pumpaerosoles.

Der Wirkstoff kommt vorzugsweise mit den bereits oben genannten Antioxidantien stabilisiert zum Einsatz. Im Falle eines offenen Systems, wie des Pumpaerosols, bei dem Außenluft in das Behältnis gelangt, wird die Zubereitung vorzugsweise konserviert, zum Beispiel durch Zusatz von Parabenen oder anderen geeigneten Konservierungsmitteln.

Das in beschriebener Weise einsetzbar wäßrige Wirkstoffsystem besteht aus:

0,01 - 50 Gew.% Wirkstoff

1 - 30 Gew.% Emulgator und/oder Lösungsvermittler

ad 100 Gew.% Wasser und Trägergas.

Besonders geeignet sind Systeme aus:

0,05 - 20 Gew.% Wirkstoff

5 - 30 Gew.% Emulgator und/oder Lösungsvermittler

ad 100 Gew.% Wasser und Trägergas.

Vorteilhaft einzusetzen sind Systeme aus:

0,1 - 10 Gew.% Wirkstoff

5 - 25 Gew.% Emulgator und/oder Lösungsvermittler

ad 100 Gew.% Wasser und Trägergas.

Insbesondere Systeme aus:

0,5 - 5 Gew.% Wirkstoff

10 - 25 Gew.% Emulgator und/oder Lösungsvermittler

ad 100 Gew.% Wasser und Trägergas.

Ein nicht beschränkendes Beispiel für ein erfindungsgemäß anzuwendendes Wirkstoffsystem wird nachstehend offenbart:

| | |
|---|---|
| Retinolpalmitat ölig (1 Mio. I.E./g) stabilisiert mit BHA/BHT | 1,0 Gew.% |
| Emulgator (Cremophor RH 40 (BASF)) | 22,0 Gew.% |
| 1,2-Propylenglykol | 2,0 Gew.% |
| Wasser und Trägergas | ad 100,0 Gew.% |

Die erfindungsgemäße pharmazeutische Zubereitung kann vorteilhaft angewandt werden zur Vorbeugung und Behandlung von Funktionseinschränkungen, Erkrankungen und krankhaften Veränderungen an den Schleimhäuten des Tracheo-Bronchialtraktes von Mensch und Tier, insbesondere am Respirationsepithel, den Epithelien des Nasen-Rachen-Raumes.

Zu den durch die Erfindung beeinflußbaren Funktionseinschränkungen, Erkrankungen und krankhaften Veränderungen gehören im besonderen Maße zelluläre Differenzierungsstörungen der Schleimhäute des Tracheo-Bronchialtraktes, Plattenepithel-Metaplasien unabhängig von der Genese, neoplastische Veränderungen, eingeschränkte Aktivität des Flimmerepithels, Dysfunktion schleimbildender Zellen unabhängig von der Genese.

Somit sind die erfindungsgemäßen pharmazeutischen Zubereitungen unter anderem geeignet zur Therapie bzw. als Adjuvans bei der Therapie von Bronchialcarcinomen, akuten und chronischen Bronchitiden, akuten und chronischen Funktionseinschränkungen infolge einer durch das Einatmen schleimhautschädigender Staube und Gase aufgetretener Beeinträchtigung des Tracheobronchial-Epithels, bronchiopulmonaler Dysplasie von Neugeborenen und des Kartagener-Syndroms.

Die erforderliche Tagesgabe liegt im Regelfall zwischen 100 und 50.000 I.E. Vitamin A bzw. Vitamin-A-Äquivalentdosis, die vorbehaltlich der toxikologischen Absicherung bezüglich systemischer und sonstiger Nebenwirkungen in 1 bis 10 Einzeldosen zu 100 - 5.000 I.E. Vitamin-A-Aktivität bzw. Vitamin-A-Äquivalentdosis in entsprechenden Intervallen verabreicht werden. Abhängig von der Art der behandelten Erkrankungen können aber auch höhere Einzel- und Tagesgaben verabreicht werden.

Die Erfindung weist insbesondere folgende Vorteile auf:

Die Erfindung stellt sicher, daß das Vitamin A und die anderen erfindungsgemäßen Wirkstoffe in der erfindungsgemäß gewählten Applikationsform in die Targetzellen aufgenommen werden.

Nach dem gegenwärtigen Stand der Technik müssen zur Erzielung einer Remission von Plattenepithelmetaplasien beim Menschen und beim Tier hohe bis höchste Konzentrationen an Vitamin A systemisch eingesetzt werden (100.000 - 500.000 IE/Tag über bis zu 60 Tage). Es konnte damit bisher nur lückenhaft dargestellt werden, daß diese nicht durch Vitamin-A-Mangel bedingte Plattenepithelmetaplasie auch quantitativ in das schleimsezernierende Ursprungsepithel rückgeführt wird und daß die metaplastisch veränderte Zelle das Vitamin auch aufnehmen kann.

Dies insbesondere deshalb, da die metaplastisch veränderte Zelle nicht mehr über Rezeptoren zur Aufnahme des Vitamin-RBP-Komplexes verfügt. Es war also nicht zu erwarten, daß eine topische Zufuhr von Vitamin A ohne Bindungsprotein durch die Zelle aufgenommen werden kann und für metabolische Aufgaben verwendet werden kann um somit eine Reversibilität der Plattenepithelmetaplasie zu bewirken.

Erfindungsgemäß konnte gezeigt werden, daß

1. Vitamin A, z.B. in Form seiner langkettigen Fettsäureester in die Zellen der Respirationsschleimhaut ohne Vermittlung von Rezeptoren und/oder Bindeproteinen nach topischer Zufuhr aufgenommen wird.

2. Das aufgenommene Vitamin in eine metabolisch aktivierbare Form übergeführt wird.

3. Das squamös metaplastisch veränderte Epithel der Respirationsschleimhaut nach topischer Zufuhr des Vitamins wieder in sein Ursprungsepithel rücküberführt werden kann.

4. Die zur Erreichung ausreichender d.h. wirksamer lokaler Vitamin A Konzentrationen im Respirationsepithel notwendigen Dosierungen bei Verwendung des Aerosols weit unter denen bei systemischer Zufuhr liegen.

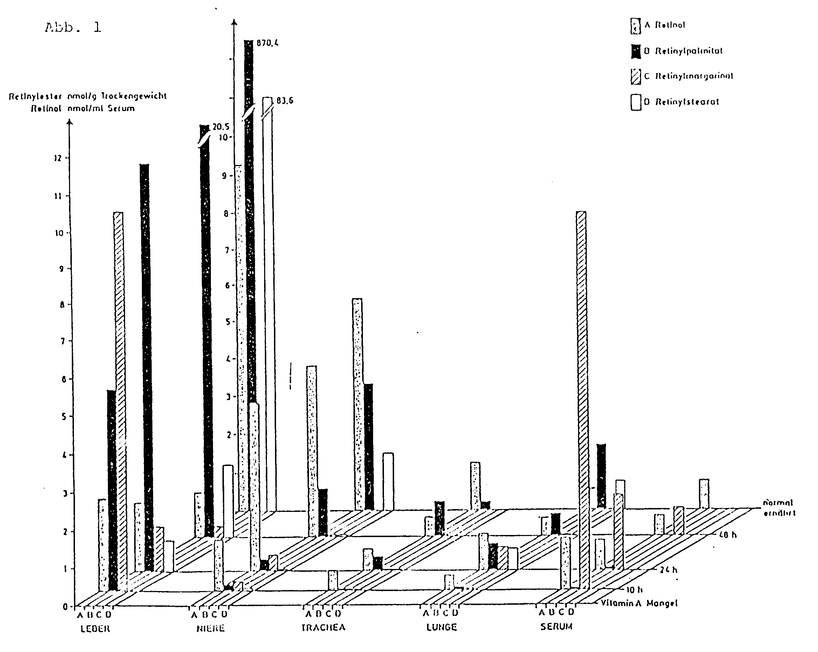

Dazu wurden die folgenden Versuche durchgeführt, die anhand der anliegenden Abbildungen 1, 2a, 2b, und 3 näher erläutert werden:

Die Abbildungen betreffen:

Abbildung 1:

Verteilung von Retinol und Retinylestern zu verschiedenen Zeitpunkten nach parenteraler Zufuhr des Retinylmargarinates. Vitamin A-Mangel: hier haben zu Versuchsbeginn keine Derivate des Retinols in den Geweben vorgelegen, während nach 48 Stunden eine deutliche Anreicherung vor allem in Lunge und Trachea beobachtet werden kann, die besonders für die Trachea die bei Normaltieren (hintere Achse) zu messende Konzentration an gespeicherten Retinylestern übertrifft.

Abbildungen 2a und 2b:

Chromatogramm der untersuchten Vitamin A-derivate 10 Stunden nach Infusion des Retinylmargarinats (2a) und 48 Stunden nach Infusion (2b).

Abbildung 3:

Vorkommen von Retinol in verschiedenen Targetgeweben Vitamin A-mangelernährter Tiere 48 Stunden nach Infusion eines C 17 Retinylesters. Zu Beginn des Versuches wiesen diese Gewebe kein Retinol auf, so daß dieses nur durch Hydrolyse des C 17 Esters in die Gewebe gelangt sein kann. Insbesondere finden sich in der Leber keine weiteren Retinolvorkommen, was wiederum beweist, daß keine Umesterung und Ausschleusung über die Leber in quantitativ bedeutsamen Maß stattgefunden hat. Der Nachweis des Retinol in den Zellen beweist darüber hinaus, daß die Hydrolyse der Ester in diesen Geweben stattgefunden hat.

Zu den vorstehenden Versuchen:

Zu 1:

Vitamin A-mangelernährte Ratten, bei denen keine peripheren Vitamin A-Speicher im Respirationsepithel mehr nachzuweisen waren, erhielten einen unphysiologischen (d.h. normalerweise nicht vorkommenden, aber metabolisierbaren) Retinylester in unterschiedlichen Konzentrationen durch ein implantiertes Infusionssystem über 6 Stunden zugeführt. Die Kinetik wurde über kontinuierliche Blutanalysen bestimmt, so daß nach 48 Stunden nach Versuchsbeginn Organproben entnommen werden konnten um die Verteilung der Retinylester in den Schleimhäuten des Respirationstraktes aber auch anderer Gewebe zu untersuchen. Es hatte sich gezeigt, daß der zugeführte Retinylester in die Schleimhaut aufgenommen, hydrolysiert und reverestert wird, so daß er in seiner metabolisierbar aktiven Form vorliegt (Abb. 1). Dies bedeutet also, daß eine Aufnahme des Vitamin A-Derivates ohne Bindungsproteine möglich ist und das Vitamin damit den Targetzellen zur Verfügung steht.

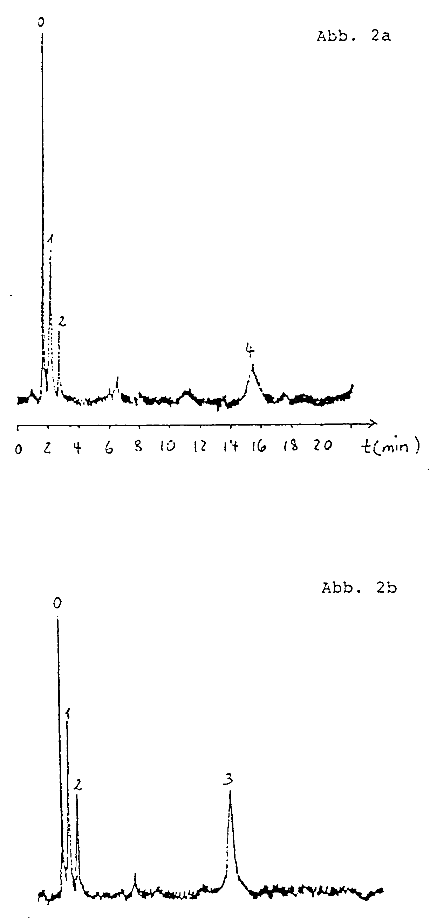

Die topische Zufuhr des C17-Retinylesters durch ein Verstäubungsverfahren über eine Trachealsonde bei mangelernährten Tieren erbrachte ein vergleichbares Resultat. Die C17-Ester werden in die Targetzellen des Respirationsepithels aufgenommen (Abb. 2a) und in eine metabolisch aktive Form überführt (Abb. 2b), wie die chromatographischen Messungen gezeigt haben.

Die Verwendung von C-17-Retinylestern bei Vitamin A-mangelernährten Tieren bietet mehrere Vorteile: Zum einen verfügen die mangelernährten Tiere über keine Vitamin A-Vorkommen in den zentralen und peripher speichernden Geweben, so daß eine Messung der Verteilung solcher Ester nach Zufuhr leicht möglich ist. Zum anderen kommt ein C17-Retinylester natürlicherweise nicht vor, so daß bei Nachweis in den Targetzellen davon ausgegangen werden kann, daß dieser Ester so in die Zelle aufgenommen wurde (siehe auch Abb. 2a). Darüber hinaus kann anhand weiterer auftretender Retinylester mit Fettsäuren unterschiedlicher Kettenlänge auf eine Hydrolyse und Reversesterung des C17-Esters geschlossen werden (siehe auch Abb. 2b).

Zu 2:

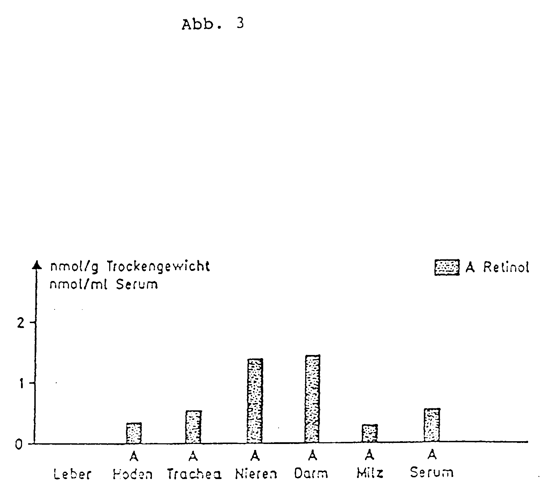

Dieser Punkt konnte in den Versuchen wie unter 1. beschrieben mit geklärt werden. Die metabolisch aktive Form des Vitamin A ist das Retinol, welches nach Hydrolyse der Retinylester in den Targetzellen an das zellulär retinolbindende Protein (CRBP) gekoppelt seine Wirkung entfalten kann. Dies geht jedoch nur, wenn das Vitamin als Ester bereits in den Zellen vorliegt, da die Aufnahme von Retinol auf dem Blutwege nach enteraler Resorption oder Freisetzung aus der Leber nur über das Transportprotein im Plasma (RBP) möglich ist. Wie in Abb. 3 gezeigt, wurde der topisch applizierte C17 Retinylester in die Zelle aufgenommen, nach Hydrolyse mit Palmitinsäure verestert, erneut hydrolysiert und liegt dann als Retinol (Abb. 3) in den Zellen vor.

Zu 3:

Bei normalernährten Ratten wurde durch chronische Inhalation von Zigarettenrauchkondensat (CSC) eine squamös metaplastische Veränderung des Tracheobronchialepithels verursacht. Insbesondere in den Bronchus 2. Art war diese metaplastische Veränderung stark ausgeprägt und zeichnet sich durch Basalzellhyperplasie und fokalen Zilienverlust aus. Diese Veränderungen waren bei allen behandelten Tieren (n = 20) deutlich ausgeprägt. Die systemische Zufuhr von 15.000 IE Vitamin A als Retinylpalmitat (2 mal pro Woche) führte nach im Mittel 22 Tagen zu einer vollständigen Rückbildung der Veränderungen, wenn die CSC-Inhalation zum Beginn der Vitamin A-Supplementierung abgesetzt worden war. Wurde während der Supplementierung weiterhin CSC inhaliert, so bildeten sich die Veränderungen später (im Mittel nach 39 Tagen) jedoch nicht vollständig zurück. Die Zilioneogenese war zwar deutlich stimuliert (gesteigerter Thymidin Labelling Index), es bleiben jedoch einige Areale in denen die Basalzellhyperplasie vollständig rückgebildet war, jedoch weiterhin geringe fokale Zilienverluste bestanden.

Bei topischer Anwendung des Vitamins durch einen Vernebler über eine Trachealsonde zeigte sich bei zweimaliger Applikation pro Tag mit je 1000 IE Vitamin A als Retinylpalmitat nach im Mittel 16 Tagen (bei Absetzen der CSC-Inhalation mit Beginn der topischen Anwendung) eine vollständige Remission der squamös metaplastischen Veränderungen, bei anhaltender CSC-Inhalation trat eine vollständige Remission nach ebenfalls 16 Tagen auf.

Damit ist gezeigt, daß die topische Anwendung von Retinylestern gegenüber der systemischen Applikation den wesentlichen Vorteil bietet, daß selbst bei bestehender Irritation des Gewebes die Regeneration eingeleitet wird und damit der Entwicklung neoplastischer Veränderungen vorbeugt. Entscheidend dabei ist aber auch, daß weitaus geringere Konzentrationen zur Erzielung einer Remission notwendig waren und somit systemisch-toxische Nebenwirkungen vermieden werden konnten.

Zu 4:

Hohe enterale Vitamin A Zufuhr (> 25.000 IE/Tag) führt zu einer chronischen Hypervitaminose A, die vor allem bei Kindern und Schwangeren zu Schädigungen führen kann. Daher ist die Supplementierung mit diesem Vitamin nur in subtoxischen Dosen unter 25.000 IE/Tag beim Menschen therapeutisch nutzbar. Da die squamös metaplastisch veränderte Zelle Retinol aus dem Plasma wegen Degeneration der membrangebundenen Retinolrezeptoren kaum aufnehmen kann, ist sie auf die Plasmaretinylester angewiesen, die ohne Rezeptoren in die Zelle gelangen können. Unter normalen Umständen kommen Retinylester im Plasma bei normaler Vitamin A-Zufuhr nur in sehr geringer Konzentration vor, so daß nur bei enteraler Supplementierung von mehr als 100.000 IE/Tag aureichende Retinylesterplasmaspiegel erreicht werden. Die normale Konzentration von im Mittel 50 μg Retinylester/g Trockengewicht in der Respirationsschleimhaut, kann bei Ratten durch Supplementierung mit mindestens 15.000 IE Vitamin A/Tag auf 75μg/g gesteigert werden. Die Plasmaretinylesterkonzentration liegt dann bei etwa 50% der Retinolkonzentration, was beim Menschen einer täglichen Dosis von 100.000 IE Vitamin A entspricht. Wird das Vitamin topisch auf die Schleimhaut aufgebracht (1000 IE Vitamin A 2 mal täglich), so steigt die ursprüngliche Konzentration in der Schleimhaut von 50 μg/g auf 110 μg/g, ohne daß eine nennenswerte Erhöhung der Plasmaretinylesterkonzentration auftritt. Da die toxischen Nebenwirkungen hoher enteraler Vitamin A-Zufuhr mit dem Anstieg der Plasmaretinylester in Zusammenhang gebracht werden, zeigt sich bei der topischen Anwendung ein deutlicher Vorteil gegenüber der systemischen Zufuhr. Toxische Nebenwirkungen können trotz längerer Anwendung vermieden werden, da erstens niedrigere Gesamtkonzentrationen zur Erzielung einer Remission als bei enteraler Zufuhr eingesetzt werden, und eine Erhöhung der Plasmaretinylester, wie sie nach systemischer Zufuhr angestrebt sein muß, ausbleibt.

Eigene Untersuchungen haben gezeigt, daß Vitamin A in Form seiner Retinylester ohne Rezeptoren nach topischer Zufuhr durch die Zellen des Respirationsepithels aufgenommen wird und in seine metabolisch aktive Form überführt wird. Dieses Ergebnis rechtfertigte die Überprüfung der Wirksamkeit einer topischen Zufuhr auf die Reversibilität von Plattenepithelmetaplasien des Respirationsepithels. Es konnte gezeigt werden, daß bei der chronischen Vitamin A Depletierung durch topische Anwendung des Aerosols

a. die Aufnahme in die squamös metaplastisch veränderten Zellen erfolgt,

b. die squamös metaplastisch veränderten Zellen wieder in ihren ursprünglichen Phänotypus rückgeführt wurden,

c. zur Erreichung dieser Rückführung bei der topischen Anwendung subtoxische, d.h. physiologische Konzentrationen ausreichend waren, während bei der systemischen Zufuhr nur die hochdosierte Applikation erfolgreich war.

Durch Anwendung eines Vitamin-A-haltigen Aerosol-Inhalats können Plattenepithelmetaplasien, wie sie infolge akuter und chronischer Bronchitis, Vitamin-A-Mangel oder aufgrund inhalierter Carcinogene bzw. physikalischer und chemischer Reize verursacht werden, in ihren ursprünglichen Phänotypus rücküberführt werden. Dabei hat das verwendete Aerosol den Vorteil, daß mit physiologischen Dosierungen gearbeitet werden kann und eine systemische Wirkung insbesondere toxischer Art umgangen werden kann.

Vorteilhaft kann die Erfindung insbesondere angewendet werden zur Behandlung von Metaplasien der Zellen der Schleimhäute des Respirationstraktes, insbesondere des Plattenepithels.

**Patentansprüche**

**Patentansprüche für folgende Vertragsstaaten : AT, BE, CH, DE, FR, GB, IT, LI, LU, NL, SE**

1. Pharmazeutische Zubereitung,
enthaltend einen Ester des Retinols und/oder der Retinsäure als Wirkstoffe,
dadurch gekennzeichnet, daß sie in der Zubereitungsform eines Aerosol-Inhalats vorliegen.

2. Pharmazeutische Zubereitung nach Anspruch 1,
dadurch gekennzeichnet,
daß als Wirkstoffe Ester des Retinols mit physiologisch unbedenklichen Carbonsäuren und/oder Ester der Retinsäure mit physiologisch unbedenklichen Alkoholen verwendet werden.

3. Pharmazeutische Zubereitung nach Anspruch 1 oder 2,
dadurch gekennzeichnet,
daß als Wirkstoffe Retinolester von gesättigten und/oder ungesättigten Fettsäuren, insbesondere von endogen vorkommenden Fettsäuren verwendet werden.

4. Pharmazeutische Zubereitung nach einem oder mehreren der vorhergehenden Ansprüche,
dadurch gekennzeichnet,
daß als Fettsäuren Palmitinsäure und/oder Stearinsäure und/oder Ölsäure und/oder Linolsäure und/oder Linolensäure verwendet werden.

5. Pharmazeutische Zubereitung nach einem oder mehreren der vorhergehenden Ansprüche,
dadurch gekennzeichnet,
daß als Alkohole zur Herstellung der Retinsäureester einwertige und/oder mehrwertige primäre und/oder sekundäre und/oder tertiäre aliphatische und/oder alicyclische und/oder aromatische Alkohole verwendet werden.

**6.** Pharmazeutische Zubereitung nach einem oder mehreren der vorhergehenden Ansprüche, dadurch gekennzeichnet, daß als Alkohole aliphatische, einwertige, primäre Alkohole, insbesondere Methanol und/oder Ethanol und/oder Fettalkohole verwendet werden.

**7.** Pharmazeutische Zubereitung nach einem oder mehreren der vorhergehenden Ansprüche, dadurch gekennzeichnet, daß die Wirkstoffe als feste Teilchen in einem Trägergas vorliegen.

**8.** Pharmazeutische Zubereitung nach einem oder mehreren der vorhergehenden Ansprüche, dadurch gekennzeichnet, daß die Wirkstoffe in Form einer wirkstoffhaltigen Flüssigkeit im Trägergas vorliegen.

**9.** Pharmazeutische Zubereitung nach einem oder mehreren der vorhergehenden Ansprüche, dadurch gekennzeichnet, daß das Trägergas teilweise oder ganz aus einem Treibgas oder Treibgasgemisch besteht, wobei als Treibgas bzw. Treib- und Trägergas niedrigsiedende Fluorkohlenwasserstoffe und/oder Fluorchlorkohlenwasserstoffe oder verflüssigte, bei Raumtemperatur und Atmosphärendruck gasförmige Verbindungen verwendet werden, insbesondere komprimierter Stickstoff oder komprimiertes Kohlendioxid oder komprimiertes Distickstoffoxid oder komprimierte Luft verwendet werden.

**10.** Pharmazeutische Zubereitung nach einem oder mehreren der vorhergehenden Ansprüche, dadurch gekennzeichnet, daß die Wirkstoffe in nichtwäßrigen flüchtigen Lösungsmitteln gelöst sind.

**11.** Pharmazeutische Zubereitung nach einem oder mehreren der vorhergehenden Ansprüche, dadurch gekennzeichnet, daß die Wirkstoffe in der flüssigen Phase des Treibgases oder Treibgasgemisches gelöst sind.

**12.** Pharmazeutische Zubereitung nach einem oder mehreren der vorhergehenden Ansprüche, dadurch gekennzeichnet, daß die Wirkstoffe in wäßrigen Lösungsmitteln gelöst oder dispergiert sind.

**13.** Pharmazeutische Zubereitung nach einem oder mehreren der vorhergehenden Ansprüche, dadurch gekennzeichnet, daß das Lösungsmittel ein toxikologisch unbedenkliches, physiologisch verträgliches, inertes Lösungsmittel, insbesondere 1,1,2-Trichlor-1,2,2-Trifluorethan (Frigen 113) oder Trichlorfluormethan (Frigen 11) ist.

**14.** Pharmazeutische Zubereitung nach einem oder mehreren der vorhergehenden Ansprüche, dadurch gekennzeichnet, daß sie besteht aus 0,01 - 50 Gew.% Wirkstoff, 0 - 49,99 Gew.% Lösungsmittel und 50 - 99,99 Gew.% Treibgas, vorzugsweise aus 0,1 - 5 Gew.% Wirkstoff, 10 - 20 Gew.% Lösungsmittel und 75 - 89,9 Gew.% Treibgas.

**15.** Pharmazeutische Zubereitung nach einem oder mehreren der vorhergehenden Ansprüche, dadurch gekennzeichnet, daß sie besteht aus 0,17 Gew.% Wirkstoff (bezogen auf Retinolpalmitat), 19,83 Gew.% Lösungsmittel und 80 Gew. % Treibgas.

**16.** Pharmazeutische Zubereitung nach einem oder mehreren der vorhergehenden Ansprüche, dadurch gekennzeichnet,

12

daß sie besteht aus
3,9 Gew.% Wirkstoff (bezogen auf Retinolpalmitat),
20 Gew.% Lösungsmittel und
76,1 Gew.% Treibgas.

17. Pharmazeutische Zubereitung nach einem oder mehreren der vorhergehenden Ansprüche,
dadurch gekennzeichnet,
daß sie besteht aus
0,01 - 50 Gew.% Wirkstoff
1 - 30 Gew.% Emulgator und/oder Lösungsvermittler
ad 100 Gew.% Wasser und Trägergas,
vorzugsweise aus
0,01 - 10 Gew.% Wirkstoff
5 - 25 Gew.% Emulgator und/oder Lösungsvermittler
ad 100 Gew.% Wasser und Trägergas.

18. Pharmazeutische Zubereitung nach einem oder mehreren der vorhergehenden Ansprüche,
dadurch gekennzeichnet,
daß sie besteht aus
0,05 - 20 Gew.% Wirkstoff
5 - 30 Gew.% Emulgator und/oder Lösungsvermittler
ad 100 Gew.% Wasser und Trägergas,
insbesondere
0,5 - 5 Gew.% Wirkstoff
10 - 25 Gew.% Emulgator und/oder Lösungsvermittler
ad 100 Gew.% Wasser und Trägergas.

19. Pharmazeutische Zubereitung nach einem oder mehreren der vorhergehenden Ansprüche,
dadurch gekennzeichnet,
daß sie besteht aus
1,0 Gew.% Retinolpalmitat ölig (1 Mio. I.E./g) stabilisiert mit BHA/BHT
22,0 Gew.% Emulgator (Cremophor RH 40 (BASF))
2,0 Gew.% 1,2-Propylenglykol
ad 100,0 Wasser und Trägergas.

20. Verwendung eines Stoffgemisches, das besteht aus einem Ester der Retinsäure und/oder einem Ester des Retinols als Wirkstoffe, die in der Zubereitungsform eines Aerosol-Inhalats vorliegen nach einem oder mehreren der vorhergehenden Ansprüche zur Herstellung eines Arzneimittels zur inhalativen topischen Anwendung für die Vorbeugung und Behandlung von Schleimhauterkrankungen des Tracheo-Bronchialtraktes von Mensch und Tier.

21. Verwendung nach Anspruch 20 zur Herstellung eines Arzneimitels zur topischen Anwendung für die Vorbeugung und Behandlung von Funktionsanomalien, Erkrankungen und krankhaften Veränderungen an den Schleimhäuten des Tracheo-Bronchialtraktes von Mensch und Tier.

22. Verwendung nach einem oder mehreren der vorhergehenden Ansprüche zur Herstellung eines Arzneimittels zur topischen Anwendung für die Vorbeugung und Behandlung von zellulären Differenzierungsstörungen der Schleimhäute des Tracheo-Bronchialtraktes, Plattenepithel-Metaplasien unabhängig von der Genese, neoplastischen Veränderungen, eingeschränkter Aktivität des Flimmerepithels und Dysfunktion schleimbildender Zellen unabhängig von der Genese.

23. Verwendung nach einem oder mehreren der vorhergehenden Ansprüche zur Herstellung eines Arzneimittels zur topischen Anwendung für die Therapie oder als topisches Adjuvans bei der Therapie von Bronchialcarcinomen, akuten und chronischon Bronchitiden, akuten und chronischon Funktionseinschränkungen infolge einer durch das Einatmen schleimhautschädigender Staube oder Gase aufgetretener Beeinträchtigungen des Tracheobronchial-Epithels, bronchiopulmonaler Dysplasie von Neugeborenen und des Kartagener-Syndroms.

**Patentansprüche für folgende Vertragsstaaten : ES, GR**

1. Verfahren zur Herstellung einer Ester des Retinols und/oder Ester der Retinsäure als Wirkstoffe enthaltenden pharmazeutischen Zubereitung, wobei die Wirkstoffe zusammen mit üblichen Zusatz- und/oder Hilfsstoffen in die Zubereitungsform eines Aerosol-Inhalats gebracht werden.

2. Verfahren nach Anspruch 1, dadurch gekennzeichnet, daß als Wirkstoffe Ester des Retinols mit physiologisch unbedenklichen Carbonsäuren und/oder Ester der Retinsäure mit physiologisch unbedenklichen Alkoholen verwendet werden.

3. Verfahren nach Anspruch 1 oder 2, dadurch gekennzeichnet, daß als Wirkstoffe Retinolester von gesättigten und/oder ungesättigten Fettsäuren, insbesondere von endogen vorkommenden Fettsäuren verwendet werden.

4. Verfahren nach einem oder mehreren der vorhergehenden Ansprüche, dadurch gekennzeichnet, daß als Fettsäuren Palmitinsäure und/oder Stearinsäure und/oder Ölsäure und/oder Linolsäure und/oder Linolensäure verwendet werden.

5. Verfahren nach einem oder mehreren der vorhergehenden Ansprüche, dadurch gekennzeichnet, daß als Alkohole zur Herstellung der Retinsäureester einwertige und/oder mehrwertige primäre und/oder sekundäre und/oder tertiäre aliphatische und/oder alicyclische und/oder aromatische Alkohole verwendet werden.

6. Verfahren nach einem oder mehreren der vorhergehenden Ansprüche, dadurch gekennzeichnet, daß als Alkohole aliphatische, einwertige, primäre Alkohole, insbesondere Methanol und/oder Ethanol und/oder Fettalkohole verwendet werden.

7. Verfahren nach einem oder mehreren der vorhergehenden Ansprüche, dadurch gekennzeichnet, daß die Wirkstoffe als feste Teilchen in einem Trägergas vorliegen.

8. Verfahren nach einem oder mehreren der vorhergehenden Ansprüche, dadurch gekennzeichnet, daß die Wirkstoffe in Form einer wirkstoffhaltigen Flüssigkeit im Trägergas vorliegen.

9. Verfahren nach einem oder mehreren der vorhergehenden Ansprüche, dadurch gekennzeichnet, daß das Trägergas teilweise oder ganz aus einem Treibgas oder Treibgasgemisch besteht, wobei als Treibgas bzw. Treib- und Trägergas niedrigsiedende Fluorkohlenwasserstoffe und/oder Fluorchlorkohlenwasserstoffe oder verflüssigte, bei Raumtemperatur und Atmosphärendruck gasförmige Verbindungen verwendet werden, insbesondere komprimierter Stickstoff oder komprimiertes Kohlendioxid oder komprimiertes Distickstoffoxid oder komprimierte Luft verwendet werden.

10. Verfahren nach einem oder mehreren der vorhergehenden Ansprüche, dadurch gekennzeichnet, daß die Wirkstoffe in nichtwäßrigen flüchtigen Lösungsmitteln gelöst sind.

11. Verfahren nach einem oder mehreren der vorhergehenden Ansprüche, dadurch gekennzeichnet, daß die Wirkstoffe in der flüssigen Phase des Treibgases oder Treibgasgemisches gelöst sind.

**12.** Verfahren nach einem oder mehreren der vorhergehenden Ansprüche,
dadurch gekennzeichnet,
daß die Wirkstoffe in wäßrigen Lösungsmitteln gelöst oder dispergiert sind.

**13.** Verfahren nach einem oder mehreren der vorhergehenden Ansprüche,
dadurch gekennzeichnet,
daß das Lösungsmittel ein toxikologisch unbedenkliches, physiologisch verträgliches, inertes Lösungsmittel, insbesondere 1,1,2-Trichlor-1,2,2-Trifluorethan (Frigen[R] 113) oder Trichlorfluormethan (Frigen[R] 11) ist.

**14.** Verfahren nach einem oder mehreren der vorhergehenden Ansprüche,
dadurch gekennzeichnet,
daß sie besteht aus
0,01 - 50 Gew.% Wirkstoff,
0 - 49,99 Gew.% Lösungsmittel und
50 - 99,99 Gew.% Treibgas, vorzugsweise aus
0,1 - 5 Gew.% Wirkstoff,
10 - 20 Gew.% Lösungsmittel und
75 - 89,9 Gew.% Treibgas.

**15.** Verfahren nach einem oder mehreren der vorhergehenden Ansprüche,
dadurch gekennzeichnet,
daß sie besteht aus
0,17 Gew.% Wirkstoff (bezogen auf Retinolpalmitat),
19,83 Gew.% Lösungsmittel und
80 Gew. % Treibgas.

**16.** Verfahren nach einem oder mehreren der vorhergehenden Ansprüche,
dadurch gekennzeichnet,
daß sie besteht aus
3,9 Gew.% Wirkstoff (bezogen auf Retinolpalmitat),
20 Gew.% Lösungsmittel und
76,1 Gew.% Treibgas.

**17.** Verfahren nach einem oder mehreren der vorhergehenden Ansprüche,
dadurch gekennzeichnet,
daß sie besteht aus
0,01 - 50 Gew.% Wirkstoff
1 - 30 Gew.% Emulgator und/oder Lösungsvermittler
ad 100 Gew.% Wasser und Trägergas,
vorzugsweise aus
0,01 - 10 Gew.% Wirkstoff
5 - 25 Gew.% Emulgator und/oder Lösungsvermittler
ad 100 Gew.% Wasser und Trägergas.

**18.** Verfahren nach einem oder mehreren der vorhergehenden Ansprüche,
dadurch gekennzeichnet,
daß sie besteht aus
0,05 - 20 Gew.% Wirkstoff
5 - 30 Gew.% Emulgator und/oder Lösungsvermittler
ad 100 Gew.% Wasser und Trägergas,
insbesondere
0,5 - 5 Gew.% Wirkstoff
10 - 25 Gew.% Emulgator und/oder Lösungsvermittler
ad 100 Gew.% Wasser und Trägergas.

**19.** Verfahren nach einem oder mehreren der vorhergehenden Ansprüche,
dadurch gekennzeichnet,

daß sie besteht aus

1,0 Gew.% Retinolpalmitat ölig (1 Mio. I.E./g) stabilisiert mit BHA/BHT

22,0 Gew.% Emulgator (Cremophor RH 40 (BASF))

2,0 Gew.% 1,2-Propylenglykol

ad 100,0 Wasser und Trägergas.

20. Verwendung eines Stoffgemisches, das besteht aus einem Ester der Retinsäure und/oder einem Ester des Retinols als Wirkstoffe, die in der Zubereitungsform eines Aerosol-Inhalats vorliegen, nach einem oder mehreren der vorhergehenden Ansprüche zur Herstellung eines Arzneimittels zur inhalativen topischen Anwendung für die Vorbeugung und Behandlung von Schleimhauterkrankungen des Tracheo-Bronchialtraktes von Mensch und Tier.

21. Verwendung nach Anspruch 20 zur Herstellung eines Arzneimitels zur topischen Anwendung für die Vorbeugung und Behandlung von Funktionsanomalien, Erkrankungen und krankhaften Veränderungen an den Schleimhäuten des Tracheo-Bronchialtraktes von Mensch und Tier.

22. Verwendung nach einem oder mehreren der vorhergehenden Ansprüche zur Herstellung eines Arzneimittels zur topischen Anwendung für die Vorbeugung und Behandlung von zellulären Differenzierungsstörungen der Schleimhäute des Tracheo-Bronchialtraktes, Plattenepithel-Metaplasien unabhängig von der Genese, neoplastischen Veränderungen, eingeschränkter Aktivität des Flimmerepithels und Dysfunktion schleimbildender Zellen unabhängig von der Genese.

23. Verwendung nach einem oder mehreren der vorhergehenden Ansprüche zur Herstellung eines Arzneimittels zur topischen Anwendung für die Therapie oder als topisches Adjuvans bei der Therapie von Bronchialcarcinomen, akuten und chronischen Bronchitiden, akuten und chronischen Funktionseinschränkungen infolge einer durch das Einatmen schleimhautschädigender Staube oder Gase aufgetretener Beeinträchtigungen des Tracheobronchial-Epithels, bronchiopulmonaler Dysplasie von Neugeborenen und des Kartagener-Syndroms.

**Claims**

**Claims for the following Contracting States : AT, BE, CH, DE, FR, GB, IT, LI, LU, NL, SE**

1. Pharmaceutical preparation, comprising an ester of retinol and/or a retinoic acid ester as active substances
characterized in
that they are present as an aerosol inhalant.

2. Pharmaceutical preparation according to claim 1,
characterized in
that as active substances esters of retinol with physiologically compatible carboxylic acids and/or esters of retinoic acids with alcohols are used.

3. Pharmaceutical preparation according to claim 1 or 2,
characterized in that as active substances retinol esters of saturated and/or unsaturated fatty acids, in particular of endogenously occuring fatty acids, are used.

4. Pharmaceutical preparation according to one or more of the preceding claims,
characterized in
that as fatty acids palmitic acid and/or stearic acid and/or oleic acid and/or linoleic acid and/or linolenic acid are used.

5. Pharmaceutical preparation according to one or more of the preceding claims,
characterized in
that as alcohols for preparing the retinoic acid esters monovalent and/or multivalent primary and/or secondary and/or tertiary aliphatic and/or alicyclic and/or aromatic alcohols are used.

6. Pharmaceutical preparation according to one or more of the preceding claims,
characterized in

that as alcohols aliphatic, monovalent, primary alcohols, in particular methanol and/or ethanol and/or fatty alcohols are used.

7.  Pharmaceutical preparation according to one or more of the preceding claims, characterized in that the active substances are present as solid particles in a carrier gas.

8.  Pharmaceutical preparation according to one or more of the preceding claims, characterized in that the active substances are present in the form of active-substance-containing liquid in a carrier gas.

9.  Pharmaceutical preparation according to one or more of the preceding claims, characterized in
    that the carrier gas consists partially or completely of a propellent gas or propellent gas mixture, wherein as propellent gas or propellent and carrier gas low-boiling fluorohydrocarbons and/or fluorochlorohydrocarbons or liquefied compounds gaseous at room temperature and atmospheric pressure are used, in particular compressed nitrogen or compressed carbon dioxide or compressed dinitrogen oxide or compressed air.

10. Pharmaceutical preparation according to one or more of the preceding claims, characterized in
    that the active substances are dissolved in nonaqueous volatile solvents.

11. Pharmaceutical preparation according to one or more of the preceding claims, characterized in
    that the active substances are dissolved in the liquid phase of the propellent gas or propellent gas mixture.

12. Pharmaceutical preparation according to one or more of the preceding claims, characterized in
    that the active substances are dissolved or dispersed in aqueous solvents.

13. Pharmaceutical preparation according to one or more of the preceding claims, characterized in
    that the solvent is a toxicologically harmless physiologically compatible inert solvent, in particular 1,1,2-trichloro-1,2,2-trifluoroethane (Frigen [R] 113) or trichlorofluoromethane (Frigen [R] 11).

14. Pharmaceutical preparation according to one or more of the preceding claims, characterized in
    that it consists of 0.01 - 50% by weight active substance, 0-49.99 % by weight solvent and
    50 - 99,99 % by weight propellent gas, in particular of
    0.1-5 % by weight active substance,
    10 - 20 % by weight solvent and
    75 - 89.9 % by weight propellent gas.

15. Pharmaceutical preparation according to one or more of the preceding claims, characterized in
    that it consists of 0.17 % by weight active substance (with respect to retinol palmitate),
    19.83 % by weight solvent and
    80 % by weight propellent gas.

16. Pharmaceutical preparation according to one or more of the preceding claims, characterized in
    that it consists of 3.9 % by weight active substance (with respect to retinol palmitate),
    20 % by weight solvent and
    76.1 % by weight propellent gas.

17. Pharmaceutical preparation according to one or more of the preceding claims, characterized in
    that it consists of 0.01 - 50 % by weight by active substance

EP 0 352 412 B1

1 - 30 % by weight emulsifier and/or solutizer
ad 100 % by weight water and carrier gas, in particular of
0.01-10 % by weight active substance
5 - 25 % by weight emulsifier and/or solutizer
ad 100 % by weight water and carrier gas.

18. Pharmaceutical preparation according to one or more of the preceding claims,
characterized in
that it consists of 0.05-20 % by weight active substance
5 - 30 % by weight emulsifier and/or solutizer
ad 100 % by weight water and carrier gas, in particular
0.5 - 5 % by weight active substance
10 - 25 % by weight emulsifier and/or solutizer
ad 100 % by weight water and carrier gas.

19. Pharmaceutical preparation according to one or more of the preceding claims,
characterized in
that it consists of 1.0 % by weight retinol palmitate oily (1 million IU/g) stabilized with BHA/BHT
22.0 % by weight emulsifier (Cremophor RH 40 (BASF))
2.0 % by weight 1,2-propylene glycol
ad 100.0 % by weight water and carrier gas.

20. Use of a mixture of compounds consisting of an ester of retinoic acid and/or an ester of retinol as active substances which are present as aerosol inhalant according to one or more of the preceding claims for the manufacture of a medicament for the inhalative topical use for preventing and treating mucosal diseases of the tracheo-bronchial tract in humans and animals.

21. Use of a pharmaceutical preparation according to claim 20 for the manufacture of a medicament for the topical use for preventing and treating functional anomalies, diseases and pathological changes in the mucous membranes of the tracheo-bronchial tract of humans and animals.

22. Use of a pharmaceutical preparation according to one or more of the preceding claims for the manufacture of a medicament for the topical use for preventing and treatment of cellular differentiation disturbances of the mucous membranes of the tracheo-bronchial tract, squamous metaplasia irrespective of the genesis, neoplastic changes, restricting activity of the ciliary epithelium and dysfunction of mucigenous cells irrespective of the genesis.

23. Use of a pharmaceutical preparation according to one or more of the preceding claims for the manufacture of a medicament for the topical use for therapy or as topical adjuvant in the therapy of bronchial carcinomas, acute and chronic bronchitis, acute and chronic functional disturbances due to impairment of the tracheo-bronchial epithelium following inhalation of dusts and gazes damaging the mucous membranes, bronchopulmonary dysplasia of newborn children and the Kartagener syndrome.

**Claims for the following Contracting States : ES, GR**

1. A process for manufacturing a pharmaceutical preparation comprising an ester of retinol and/or a retinoic acid ester as active substances, wherein the active substances are formulated together with usual additives or auxiliaries as an aerosol-inhalant.

2. A process according to claim 1,
characterized in
that as active substances esters of retinol with physiologically compatible carboxylic acids and/or esters of retinoic acids with alcohols are used.

3. A process according to claim 1 or 2,
characterized in that as active substances retinol esters of saturated and/or unsaturated fatty acids, in particular of endogenously occuring fatty acids, are used.

18

EP 0 352 412 B1

4. A process according to one or more of the preceding claims,
characterized in
that as fatty acids palmitic acid and/or stearic acid and/or oleic acid and/or linoleic acid and/or linolenic acid are used.

5. A process according to one or more of the preceding claims,
characterized in
that as alcohols for preparing the retinoic acid esters monovalent and/or multivalent primary and/or secondary and/or tertiary aliphatic and/or alicyclic and/or aromatic alcohols are used.

6. A process according to one or more of the preceding claims,
characterized in
that as alcohols aliphatic, monovalent, primary alcohols, in particular methanol and/or ethanol and/or fatty alcohols are used.

7. A process according to one or more of the preceding claims,
characterized in that the active substances are present as solid particles in a carrier gas.

8. A process according to one or more of the preceding claims, characterized in that the active substances are present in the form of active-substance-containing liquid in a carrier gas.

9. A process according to one or more of the preceding claims,
characterized in
that the carrier gas consists partially or completely of a propellent gas or propellent gas mixture, wherein as propellent gas or propellent and carrier gas low-boiling fluorohydrocarbons and/or fluorochlorohydrocarbons or liquefied compounds gaseous at room temperature and atmospheric pressure are used, in particular compressed nitrogen or compressed carbon dioxide or compressed dinitrogen oxide or compressed air.

10. A process according to one or more of the preceding claims,
characterized in
that the active substances are dissolved in nonaqueous volatile solvents.

11. A process according to one or more of the preceding claims,
characterized in
that the active substances are dissolved in the liquid phase of the propellent gas or propellent gas mixture.

12. A process according to one or more of the preceding claims,
characterized in
that the active substances are dissolved or dispersed in aqueous solvents.

13. A process according to one or more of the preceding claims,
characterized in
that the solvent is a toxicologically harmless physiologically compatible inert solvent, in particular 1,1,2-trichloro-1,2,2-trifluoroethane (Frigen [R] 113) or trichlorofluoromethane (Frigen [R] 11).

14. A process according to one or more of the preceding claims,
characterized in
that it consists of 0.01 - 50% by weight active substance, 0-49.99 % by weight solvent and
50 - 99,99 % by weight propellent gas, in particular of
0.1-5 % by weight active substance,
10 - 20 % by weight solvent and
75 - 89.9 % by weight propellent gas.

15. A process according to one or more of the preceding claims,
characterized in
that it consists of 0.17 % by weight active substance (with respect to retinol palmitate),

19

19.83 % by weight solvent and
80 % by weight propellent gas.

16. A process according to one or more of the preceding claims,
characterized in
that it consists of 3.9 % by weight active substance (with respect to retinol palmitate),
20 % by weight solvent and
76.1 % by weight propellent gas.

17. A process according to one or more of the preceding claims,
characterized in
that it consists of 0.01 - 50 % by weight by active substance
1 - 30 % by weight emulsifier and/or solutizer
ad 100 % by weight water and carrier gas, in particular of
0.01-10 % by weight active substance
5 - 25 % by weight emulsifier and/or solutizer
ad 100 % by weight water and carrier gas.

18. A process according to one or more of the preceding claims,
characterized in
that it consists of 0.05-20 % by weight active substance
5 - 30 % by weight emulsifier and/or solutizer
ad 100 % by weight water and carrier gas, in particular
0.5 - 5 % by weight active substance
10 - 25 % by weight emulsifier and/or solutizer
ad 100 % by weight water and carrier gas.

19. A process according to one or more of the preceding claims,
characterized in
that it consists of 1.0 % by weight retinol palmitate oily (1 million IU/g) stabilized with BHA/BHT
22.0 % by weight emulsifier (Cremophor RH 40 (BASF))
2.0 % by weight 1,2-propylene glycol
ad 100.0 % by weight water and carrier gas.

20. Use of a mixture of compounds consisting of an ester of retinoic acid and/or an ester of retinol as active substances which are present as aerosol inhalant according to one or more of the preceding claims for the manufacture of a medicament for the inhalative topical use for preventing and treating mucosal diseases of the tracheo-bronchial tract in humans and animals.

21. Use of a pharmaceutical preparation according to claim 20 for the manufacture of a medicament for the topical use for preventing and treating functional anomalies, diseases and pathological changes in the mucous membranes of the tracheo-bronchial tract of humans and animals.

22. Use of a pharmaceutical preparation according to one or more of the preceding claims for the manufacture of a medicament for the topical use for preventing and treatment of cellular differentiation disturbances of the mucous membranes of the tracheo-bronchial tract, squamous metaplasia irrespective of the genesis, neoplastic changes, restricting activity of the ciliary epithelium and dysfunction of mucigenous cells irrespective of the genesis.

23. Use of a pharmaceutical preparation according to one or more of the preceding claims for the manufacture of a medicament for the topical use for therapy or as topical adjuvant in the therapy of bronchial carcinomas, acute and chronic bronchitis, acute and chronic functional disturbances due to impairment of the tracheo-bronchial epithelium following inhalation of dusts and gases damaging the mucous membranes, bronchopulmonary dysplasia of newborn children and the Kartagener syndrome.

**Revendications**
**Revendications pour les Etats contractants suivants : AT, BE, CH, DE, FR, GB, IT, LI, LU, NL, SE**

1. Préparation pharmaceutique contenant un ester du rétinol et/ou de l'acide rétinoïque en tant que substances actives, caractérisée en ce qu'elle se présente sous la forme de préparation d'un produit d'inhalation en aérosol.

2. Préparation pharmaceutique suivant la revendication 1, caractérisée en ce que, comme substances actives, on utilise un ester du rétinol, comportant des acides carboxyliques inoffensifs sur le plan physiologique, et/ou un ester de l'acide rétinoïque comportant des alcools inoffensifs sur le plan physiologique.

3. Préparation pharmaceutique suivant l'une des revendications 1 et 2, caractérisée en ce que, comme substances actives, on utilise un ester du rétinol et d'acides gras saturés et/ou insaturés, notamment d'acides gras d'origine endogène.

4. Préparation pharmaceutique suivant une ou plusieurs des revendications précédentes, caractérisée en ce que, comme acides gras, on utilise l'acide palmitique et/ou l'acide stéarique et/ou l'acide oléique et/ou l'acide linoléique et/ou l'acide linolénique.

5. Préparation pharmaceutique suivant une ou plusieurs des revendications précédentes, caractérisée en ce que, comme alcools servant à la préparation de l'ester d'acide rétinoïque, on utilise des monoalcools et/ou des polyalcools, aliphatiques et/ou alicycliques et/ou aromatiques, primaires et/ou secondaires et/ou tertiaires.

6. Préparation pharmaceutique suivant une ou plusieurs des revendications précédentes, caractérisée en ce que, comme alcools, on utilise des monoalcools primaires aliphatiques, notamment le méthanol et/ou l'éthanol et/ou des alcools gras.

7. Préparation pharmaceutique suivant une ou plusieurs des revendications précédentes, caractérisée en ce que les substances actives sont présentes sous la forme de particules solides dans un véhicule gazeux.

8. Préparation pharmaceutique suivant une ou plusieurs des revendications précédentes, caractérisée en ce que les substances actives sont présentes dans le véhicule gazeux sous la forme d'un liquide contenant des substances actives.

9. Préparation pharmaceutique suivant une ou plusieurs des revendications précédentes, caractérisée en ce que le véhicule gazeux est partiellement ou totalement constitué d'un gaz propulseur ou d'un mélange de gaz propulseurs et en ce que, comme gaz propulseur ou véhicule gazeux propulseur, on utilise des composés fluorohydrocarbonés et/ou des composés fluorochlorohydrocarbonés à bas point d'ébullition ou des composés, qui sont gazeux à la température ambiante et la pression atmosphérique, à l'état liquéfié, notamment de l'azote comprimé ou de l'anhydride carbonique comprimé ou du protoxyde d'azote comprimé ou de l'air comprimé.

10. Préparation pharmaceutique suivant une ou plusieurs des revendications précédentes, caractérisée en ce que les substances actives sont dissoutes dans des solvants volatils non aqueux.

11. Préparation pharmaceutique suivant une ou plusieurs des revendications précédentes, caractérisée en ce que les substances actives sont dissoutes dans la phase liquide du gaz propulseur ou du mélange de gaz propulseurs.

12. Préparation pharmaceutique suivant une ou plusieurs des revendications précédentes, caractérisée en ce que les substances actives sont dissoutes ou mises en dispersion dans des solvants aqueux.

13. Préparation pharmaceutique suivant une ou plusieurs des revendications précédentes, caractérisée en ce que' le solvant est un solvant inerte compatible sur le plan physiologique et inoffensif sur le plan toxicologique, notamment le 1,1,2-trichloro-1,2,2-trifluoroéthane (Frigen 113) ou le trichlorofluorométhane (Frigen 11).

14. Préparation pharmaceutique suivant une ou plusieurs des revendications précédentes, caractérisée en ce qu'elle est constituée de :

0,01-50 % en poids de substance active
0-49,99 % en poids de solvant et
50-99,99 % en poids de gaz propulseur, de préférence de :
0,1-5 % en poids de substance active,
10-20 % en poids de solvant et
75-89,9 % en poids de gaz propulseur.

15. Préparation pharmaceutique suivant une ou plusieurs des revendications précédentes, caractérisée en ce qu'elle est constituée de :

0,17 % en poids de substance active (rapporté à du palmitate de rétinol),
19,83 % en poids de solvant et
80 % en poids de gaz propulseur.

16. Préparation pharmaceutique suivant une ou plusieurs des revendications précédentes, caractérisée en ce qu'elle est constituée de :

3,9 % en poids de substance active (rapporté à du palmitate de rétinol),
20 % en poids de solvant et
76,1 % en poids de gaz propulseur.

17. Préparation pharmaceutique suivant une ou plusieurs des revendications précédentes, caractérisée en ce qu'elle est constituée de :

0,01-50 % en poids de substance active,
1-30 % en poids d'agent émulsionnant et/ou d'agent solubilisant et le complément à 100 % en poids d'eau et de véhicule gazeux,
de préférence de :
0,01-10 % en poids de substance active,
5-25 % en poids d'agent émulsionnant et/ou d'agent solubilisant et
le complément à 100 % en poids d'eau et de véhicule gazeux.

18. Préparation pharmaceutique suivant une ou plusieurs des revendications précédentes, caractérisée en ce qu'elle est constituée de :

0,05-20 % en poids de substance active,
5-30 % en poids d'agent émulsionnant et/ou d'agent solubilisant et
le complément à 100 % en poids d'eau et de véhicule gazeux,
notamment de :
0,5-5 % en poids de substance active,
10-25 % en poids d'agent émulsionnant et/ou d'agent solubilisant et
le complément à 100 % en poids d'eau et de véhicule gazeux.

19. Préparation pharmaceutique suivant une ou plusieurs des revendications précédentes, caractérisée en ce qu'elle est constituée de :

1,0 % en poids de palmitate de rétinol huileux (1 Mio. I.E./g), stabilisé par BHA/BHT,
22,0 % en poids d'agent émulsionnant (Cremophor RH 40 (BASF)),
2,0 % en poids de 1,2-propylèneglycol et
le complément à 100 % d'eau et de véhicule gazeux.

20. Utilisation d'un mélange de substances qui est constitué d'un ester de l'acide rétinoïque et/ou d'un ester du rétinol en tant que substances actives qui sont présentes dans la forme de préparation d'un produit d'inhalation en aérosol, suivant une ou plusieurs des revendications précédentes, pour la préparation d'un médicament servant à l'application topique par inhalation en vue de la prévention et du traitement des maladies des muqueuses du tractus trachéo-bronchique de l'homme et de l'animal.

21. Utilisation suivant la revendication 20 pour la préparation d'un médicament servant à l'application topique en vue de la prévention et du traitement d'anomalies fonctionnelles, maladies et modifications

EP 0 352 412 B1

pathologiques se présentant sur les muqueuses du tractus trachéo-bronchique de l'homme et de l'animal.

22. Utilisation suivant une ou plusieurs des revendications précédentes pour la préparation d'un médicament servant à l'application topique en vue de la prévention et du traitement de troubles de différenciation cellulaire des muqueuses du tractus trachéo-bronchique, de métaplasies de l'épithélium lisse de type non congénital, de modifications néoplasiques, d'une activité limitée de l'épithélium vibratile et d'un dysfonctionnement, de type non congénital, de cellules formatrices de muqueuse.

23. Utilisation suivant une ou plusieurs des revendications précédentes pour la préparation d'un médicament servant à l'application topique en vue de la thérapie ou comme adjuvant topique dans la thérapie de carcinomes bronchiques, de bronchites aiguës et chroniques, de restrictions fonctionnelles aiguës et chroniques dues à une atteinte de l'épithélium trachéo-bronchique provenant de l'inhalation de poussières ou de gaz nocifs pour la muqueuse, d'une dysplasie broncho-pulmonaire des nouveaux-nés et du syndrome de Kartagener.

**Revendications pour les Etats contractants suivants : ES, GR**

1. Procédé de préparation d'un ester du rétinol et/ou d'un ester de l'acide rétinoïque en tant que préparation pharmaceutique contenant des substances actives, les substances actives étant introduites, avec des additifs et/ou substances auxiliaires courants, dans la forme de préparation d'un produit d'inhalation en aérosol.

2. Procédé suivant la revendication 1, caractérisé en ce que, comme substances actives, on utilise un ester du rétinol, comportant des acides carboxyliques inoffensifs sur le plan physiologique, et/ou un ester de l'acide rétinoïque comportant des alcools inoffensifs sur le plan physiologique.

3. Procédé suivant l'une des revendications 1 et 2, caractérisé en ce que, comme substances actives, on utilise un ester du rétinol et d'acides gras saturés et/ou insaturés, notamment d'acides gras d'origine endogène.

4. Procédé suivant une ou plusieurs des revendications précédentes, caractérisé en ce que, comme acides gras, on utilise l'acide palmitique et/ou l'acide stéarique et/ou l'acide oléique et/ou l'acide linoléique et/ou l'acide linolénique.

5. Procédé suivant une ou plusieurs des revendications précédentes, caractérisé en ce que, comme alcools servant à la préparation de l'ester d'acide rétinoïque, on utilise des monoalcools et/ou des polyalcools, aliphatiques et/ou alicycliques et/ou aromatiques, primaires et/ou secondaires et/ou tertiaires.

6. Procédé suivant une ou plusieurs des revendications précédentes, caractérisé en ce que, comme alcools, on utilise des monoalcools primaires aliphatiques, notamment le méthanol et/ou l'éthanol et/ou des alcools gras.

7. Procédé suivant une ou plusieurs des revendications précédentes, caractérisé en ce que les substances actives sont présentes sous la forme de particules solides dans un véhicule gazeux.

8. Procédé suivant une ou plusieurs des revendications précédentes, caractérisé en ce que les substances actives sont présentes dans le véhicule gazeux sous la forme d'un liquide contenant des substances actives.

9. Procédé suivant une ou plusieurs des revendications précédentes, caractérisé en ce que le véhicule gazeux est partiellement ou totalement constitué d'un gaz propulseur ou d'un mélange de gaz propulseurs et en ce que, comme gaz propulseur ou véhicule gazeux propulseur, on utilise des composés fluorohydrocarbonés et/ou des composés fluorochlorohydrocarbonés à bas point d'ébullition ou des composés, qui sont gazeux à la température ambiante et la pression atmosphérique, à l'état liquéfié, notamment de l'azote comprimé ou de l'anhydride carbonique comprimé ou du protoxyde d'azote comprimé ou de l'air comprimé.

23

**10.** Procédé suivant une ou plusieurs des revendications précédentes, caractérisé en ce que les substances actives sont dissoutes dans des solvants volatils non aqueux.

**11.** Procédé suivant une ou plusieurs des revendications précédentes, caractérisé en ce que les substances actives sont dissoutes dans la phase liquide du gaz propulseur ou du mélange de gaz propulseurs.

**12.** Procédé suivant une ou plusieurs des revendications précédentes, caractérisé en ce que les substances actives sont dissoutes ou mises en dispersion dans des solvants aqueux.

**13.** Procédé suivant une ou plusieurs des revendications précédentes, caractérisé en ce que le solvant est un solvant inerte compatible sur le plan physiologique et inoffensif sur le plan toxicologique, notamment le 1,1,2-trichloro-1,2,2-trifluoroéthane (Frigen® 113) ou le trichlorofluorométhane (Frigen® 11).

**14.** Procédé suivant une ou plusieurs des revendications précédentes, caractérisé en ce que la préparation pharmaceutique est constituée de :
>   0,01-50 % en poids de substance active
>   0-49,99 % en poids de solvant et
>   50-99,99 % en poids de gaz propulseur, de préférence de :
>   0,1-5 % en poids de substance active,
>   10-20 % en poids de solvant et
>   75-89,9 % en poids de gaz propulseur.

**15.** Procédé suivant une ou plusieurs des revendications précédentes, caractérisé en ce que la préparation pharmaceutique est constituée de :
>   0,17 % en poids de substance active (rapporté à du palmitate de rétinol),
>   19,83 % en poids de solvant et
>   80 % en poids de gaz propulseur.

**16.** Procédé suivant une ou plusieurs des revendications précédentes, caractérisé en ce que la préparation pharmaceutique est constituée de :
>   3,9 % en poids de substance active (rapporté à du palmitate de rétinol),
>   20 % en poids de solvant et
>   76,1 % en poids de gaz propulseur.

**17.** Procédé suivant une ou plusieurs des revendications précédentes, caractérisé en ce que la préparation pharmaceutique est constituée de :
>   0,01-50 % en poids de substance active,
>   1-30 % en poids d'agent émulsionnant et/ou d'agent solubilisant et
>   le complément à 100 % en poids d'eau et de véhicule gazeux,
>   de préférence de :
>   0,01-10 % en poids de substance active,
>   5-25 % en poids d'agent émulsionnant et/ou d'agent solubilisant et
>   le complément à 100 % en poids d'eau et de véhicule gazeux.

**18.** Procédé suivant une ou plusieurs des revendications précédentes, caractérisé en ce que la préparation pharmaceutique est constituée de :
>   0,05-20 % en poids de substance active,
>   5-30 % en poids d'agent émulsionnant et/ou d'agent solubilisant et
>   le complément à 100 % en poids d'eau et de véhicule gazeux,
>   notamment de :
>   0,5-5 % en poids de substance active,
>   10-25 % en poids d'agent émulsionnant et/ou d'agent solubilisant et
>   le complément à 100 % en poids d'eau et de véhicule gazeux.

**19.** Procédé suivant une ou plusieurs des revendications précédentes, caractérisé en ce que la préparation pharmaceutique est constituée de :
>   1,0 % en poids de palmitate de rétinol huileux (1 Mio. I.E./g), stabilisé par BHA/BHT,
>   22,0 % en poids d'agent émulsionnant (Cremophor RH 40 (BASF)),

24

2,0 % en poids de 1,2-propylèneglycol et
le complément à 100 % d'eau et de véhicule gazeux.

20. Utilisation d'un mélange de substances qui est constitué d'un ester de l'acide rétinoïque et/ou d'un ester du rétinol en tant que substances actives qui sont présentes dans la forme de préparation d'un produit d'inhalation en aérosol, suivant une ou plusieurs des revendications précédentes, pour la préparation d'un médicament servant à l'application topique par inhalation en vue de la prévention et du traitement des maladies des muqueuses du tractus trachéo-bronchique de l'homme et de l'animal.

21. Utilisation suivant la revendication 20 pour la préparation d'un médicament servant à l'application topique en vue de la prévention et du traitement d'anomalies fonctionnelles, maladies et modifications pathologiques se présentant sur les muqueuses du tractus trachéo-bronchique de l'homme et de l'animal.

22. Utilisation suivant une ou plusieurs des revendications précédentes pour la préparation d'un médicament servant à l'application topique en vue de la prévention cet du traitement de troubles de différenciation cellulaire des muqueuses du tractus trachéo-bronchique, de métaplasies de l'épithélium lisse de type non congénital, de modifications néoplasiques, d'une activité limitée de l'épithélium vibratile et d'un dysfonctionnement, de type non congénital, de cellules formatrices de muqueuse.

23. Utilisation suivant une ou plusieurs des revendications précédentes pour la préparation d'un médicament servant à l'application topique en vue de la thérapie ou comme adjuvant topique dans la thérapie de carcinomes bronchiques, de bronchites aiguës et chroniques, de restrictions fonctionnelles aiguës et chroniques dues à une atteinte de l'épithélium trachéo-bronchique provenant de l'inhalation de poussières ou de gaz nocifs pour la muqueuse, d'une dysplasie broncho-pulmonaire des nouveaux-nés et du syndrome de Kartagener.

Abb. 1

Abb. 2a

Abb. 2b

Abb. 3